(19) Europäisches Patentamt
European Patent Office
Office européen des brevets

(11)  **EP 4 209 457 A1**

(12)  **EUROPEAN PATENT APPLICATION**
published in accordance with Art. 153(4) EPC

(43) Date of publication:
**12.07.2023  Bulletin 2023/28**

(51) International Patent Classification (IPC):
*C01F 7/00* (2022.01)          *A61K 9/14* (2006.01)
*A61K 31/198* (2006.01)        *A61K 38/22* (2006.01)
*A61K 45/00* (2006.01)         *A61K 47/52* (2017.01)
*A61P 3/02* (2006.01)          *A61P 31/04* (2006.01)
*A61P 35/00* (2006.01)         *A61P 43/00* (2006.01)
*B82Y 5/00* (2011.01)          *B82Y 30/00* (2011.01)

(21) Application number: 21864231.2

(22) Date of filing: 26.08.2021

(52) Cooperative Patent Classification (CPC):
A61K 9/14; A61K 31/198; A61K 38/22;
A61K 45/00; A61K 47/52; A61P 3/02; A61P 31/04;
A61P 35/00; A61P 43/00; B82Y 5/00; B82Y 30/00;
C01F 7/00

(86) International application number:
PCT/JP2021/031394

(87) International publication number:
WO 2022/050173 (10.03.2022 Gazette 2022/10)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  01.09.2020  JP 2020147037

(71) Applicant: SETOLAS Holdings, Inc.
Takamatsu-shi, Kagawa 760-0026 (JP)

(72) Inventors:
• NISHIYA, Ken
  Kita-gun, Kagawa 761-0705 (JP)
• IWAMOTO, Yoshihito
  Sakaide-shi, Kagawa 762-0012 (JP)
• TAKABATAKE, Harumi
  Sakaide-shi, Kagawa 762-0012 (JP)

(74) Representative: **Vossius & Partner
Patentanwälte Rechtsanwälte mbB
Siebertstraße 3
81675 München (DE)**

(54)  **SURFACE-TREATED HYDROTALCITE, SUSPENSION OF SAME, AND FUNCTIONAL MOLECULE DELIVERY SYSTEM USING SAME**

(57)  This surface-treated hydrotalcite is obtained by surface-treating a hydrotalcite having a compositional makeup represented by formula (1) and satisfies (A)-(C). This hydrotalcite has excellent dispersibility.

$$(M^{2+})_{(1-x)}(M^{3+})_x(OH)_2(A^{n-})_{x/n} \cdot mH_2O \qquad (1)$$

(Wherein, respective signs in formula (1) are as defined in the claims.)
(A) The proportion of a surface-treating agent is 20-50 mass%.
(B) The average width of primary particles is 5-120 nm.
(C) The monodispersity is 75% or more.

**(Cont. next page)**

EP 4 209 457 A1

# FIGURE 1

Width of primary particles ($W_1$)

## Description

### TECHNICAL FIELD

The present invention relates to a surface-treated hydrotalcite and a suspension thereof, as well as a functional molecule delivery system using the same.

### BACKGROUND ART

An effect called EPR (Enhanced Permeability and Retention) has been observed in the vicinity of tumor tissue. This refers to a phenomenon where nanoparticles tend to accumulate in tumor tissue because gaps of about 200 nm are generated between vascular endothelial cells around tumor tissue due to increased angiogenesis, which allows nanoparticles to leak out of the vessels (enhanced permeability), and also because lymph vessels are underdeveloped in tumor tissue, which makes it difficult for foreign substances to be excreted (enhanced retention) (Non-Patent Literature 1).

In recent years, there have been an increasing number of technologies offered to deliver drugs to tumor tissue using the EPR effect. Candidate materials that can be used for such drug delivery technologies include hydrotalcites, which are being developed for nanosizing and as drug carriers because of their ability to incorporate anionic drugs between their layers (Patent Literatures 1 and 2).

However, although nano-sized hydrotalcite particles exhibit good dispersibility in water, they aggregate and cannot maintain their dispersibility in the presence of salts, such as in biological conditions. In addition, when functional molecules are intercalated between layers of hydrotalcite, agglomeration occurs due to fusion of particle surfaces, making it difficult to maintain their nanoparticle size.

Patent Literature 1 discloses a hydrotalcite whose primary particles have an average width of between 5 nm and 200 nm. However, according to this document, the particle size of the hydrotalcite was measured after the hydrotalcite was added to ethanol and subjected to ultrasonication for facilitating dispersion. Therefore, the particle diameter of hydrotalcite described in this document cannot correspond to the diameter of hydrotalcite suspended in a solution containing salts such as saline solution, nor is it the diameter of hydrotalcite suspended without carrying out any treatment for facilitating dispersion.

Patent Literature 2 discloses a hydrotalcite with a volume-weighted average particle diameter of 500 nm or less before functional molecules are intercalated into their layers. However, the present inventors' follow-up test has revealed that in the technology described in this document, agglomeration of hydrotalcite occurs when functional molecules are intercalated. Therefore, it is not deemed that the particle size described in this document can be maintained even after the introduction of functional molecules to between layers of the hydrotalcite.

### LIST OF CITATIONS

#### Non-Patent Literature

[Non-Patent Literature 1] Matsumura et al., Cancer Research, (1986), Vol.46, No. 12, p.6387-6392

#### Patent Literature

[Patent Literature 1] WO2018/169019 A
[Patent Literature 2] JP2007-538098 A

### SUMMARY OF INVENTION

### PROBLEM TO BE SOLVED BY THE INVENTION

The problem to be addressed by the present invention is to provide a hydrotalcite with excellent dispersibility.

### MEANS TO SOLVE THE PROBLEM

As a result of diligent research in view of the aforementioned problem, the present inventors have found that the dispersibility of a hydrotalcite can be improved remarkably by surface-treating the hydrotalcite with a larger amount of surface treatment agent than in the prior art and adjusting the average width of the primary particles within a certain range, whereby the above problem can be solved. Based on this finding, the present inventors have completed the present invention.

Specifically, the present invention encompasses the following aspects.

[Aspect 1] A surface-treated hydrotalcite derived by treating a hydrotalcite having a compositional makeup represented by formula (1) below with a surface treatment agent, wherein the surface-treated hydrotalcite satisfies the requirements (A) to (C) below.

[Formula 1]

$$(M^{2+})_{(1-x)}(M^{3+})_x(OH)_2(A^{n-})_{x/n} \cdot mH_2O \qquad (1)$$

In Formula (1), $M^{2+}$ represents at least one divalent metal cation, $M^{3+}$ represents at least one trivalent metal cation, $A^{n-}$ represents at least one n-valent anion, n represents an integer of 1 to 6, x represents a real number satisfying $0.17 \leq x \leq 0.38$, and m represents a real number satisfying $0 \leq m \leq 2$.

(A) The mass ratio of the surface treatment agent to the hydrotalcite is 20 mass % or higher but 50 mass % or lower.
(B) The average width of the primary particles is from 5 nm to 120 nm.
(C) The monodispersity represented by Formula (2) below is 75% or higher.

[Formula 2]

$$\text{Monodispersity (\%)} = \frac{\text{Average width of primary particles}}{\text{Average diameter } (D_{50}) \text{ in suspension}} \times 100 \qquad (2)$$

[Aspect 2] The surface-treated hydrotalcite according to Aspect 1, wherein the average particle diameter $(D_{50})$ of the hydrotalcite in a suspension is 120 nm or less.

[Aspect 3] The surface-treated hydrotalcite according to Aspect 1 or 2, wherein the surface treatment agent is at least one selected from the group consisting of anionic surfactants, cationic surfactants, phosphoric acid ester-based treatment agents, silane coupling agents, titanate coupling agents, aluminum coupling agents, silicone-based treatment agents, silicates, liquid glass, and polyanionic polymer compounds.

[Aspect 4] The surface-treated hydrotalcite according to any one of Aspects 1 to 3, further comprising a functional molecule between layers.

[Aspect 5] The surface-treated hydrotalcite according to Aspect 4, wherein the functional molecule is at least one selected from the group consisting of drugs, antibiotics, antimicrobials, amino acids, peptides, proteins, therapeutic agents, hormones, enzymes, growth factors, RNAs, DNAs, and oligonucleotides.

[Aspect 6] A surface-treated hydrotalcite suspension comprising a liquid medium and a surface-treated hydrotalcite according to any one of Aspects 1 to 3 suspended in the liquid medium.

[Aspect 7] The surface-treated hydrotalcite suspension according to Aspect 6, wherein the liquid medium is deionized water or a physiological isotonic solution.

[Aspect 8] The surface-treated hydrotalcite suspension according to Aspect 6 or 7, wherein the liquid medium is a physiological isotonic solution, and the monodispersity of the surface-treated hydrotalcite in the physiological isotonic solution is 75% or higher.

[Aspect 9] The surface-treated hydrotalcite suspension according to any one of Aspects 6 to 8, further comprising a functional molecule between layers of the surface-treated hydrotalcite.

[Aspect 10] The surface-treated hydrotalcite suspension according to Aspect 9, wherein the functional molecule is at least one selected from the group consisting of drugs, antibiotics, antimicrobials, amino acids, peptides, proteins, therapeutic agents, hormones, enzymes, growth factors, RNAs, DNAs, and oligonucleotides.

[Aspect 11] A functional molecule delivery system comprising a surface-treated hydrotalcite according to any one of Aspects 1 to 5 and/or a surface-treated hydrotalcite suspension according to any one of Aspects 6 to 10.

**EFFECT OF THE INVENTION**

The present invention provides a hydrotalcite with excellent dispersibility.

**BRIEF EXPLANATION OF FIGURES**

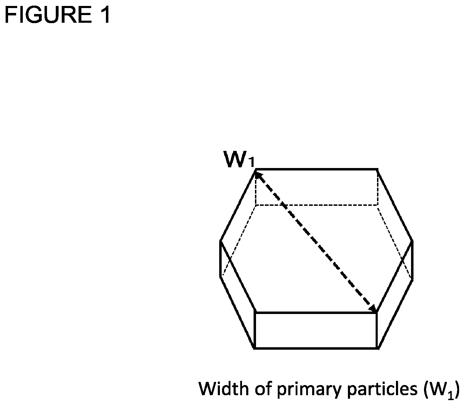

[Figure 1] Figure 1 is a schematic diagram for illustrating the width (W1) of a primary particle of hydrotalcite.

**DESCRIPTION OF EMBODIMENTS**

The present invention will be described in detail in accordance with the specific embodiments below. However, the invention is not bound by the following embodiments, but can be implemented in any form to the extent that it does not depart from the intent of the present invention.

[I. Surface-treated hydrotalcite and its suspension]

One aspect of the present invention relates to a surface-treated hydrotalcite having a specific compositional makeup and physical properties (hereinafter referred to as "the surface-treated hydrotalcite of the present invention"). The surface-treated hydrotalcite of the present invention is characterized by being derived from a hydrotalcite having a compositional makeup represented by Formula (1) below via surface treatment and also satisfying the requirements (A) to (C) below.

*Compositional makeup of the hydrotalcite:

The surface-treated hydrotalcite of the present invention is prepared by surface-treating a hydrotalcite having a compositional makeup represented by Formula (1) below (the hydrotalcite before the surface treatment may also be referred to as "the hydrotalcite of the present invention").
[Formula 3]

$$(M^{2+})_{(1-x)}(M^{3+})_x(OH)_2(A^{n-})_{x/n} \cdot mH_2O \qquad (1)$$

In Formula (1), $M^{2+}$ represents at least one divalent metal. Examples of $M^{2+}$ include $Mg^{2+}$, $Fe^{2+}$, $Zn^{2+}$, $Ca^{2+}$, $Li^{2+}$, $Ni^{2+}$, $Co^{2+}$, and $Cu^2$. Among them, one or more selected from $Mg^{2+}$ and $Zn^{2+}$ are preferred as $M^{2+}$ because of its high safety for the living body.
In Formula (1), $M^{3+}$ represents at least one trivalent metal. Examples of $M^{3+}$ include $Al^{3+}$, $Fe^{3+}$, and $Mn^{3+}$. Among them, $Al^{3+}$ is preferred as $M^{3+}$ because of its high safety for the living body.
In Formula (1), $A^{n-}$ represents at least one n-valent anion, where n represents an integer of from 1 to 6. $A^{n-}$ is mainly present between the layers of hydrotalcite (hereinafter also referred to as "interlayer anions" as appropriate), although some of it may be present outside the layers of hydrotalcite. Examples of $A^{n-}$ include one or more selected from chloride ion ($Cl^-$), nitrate ion ($NO^{3-}$), acetate ion ($CH_3COO^-$), and carbonate ion ($CO_3^{2-}$). In the following description, hydrotalcite may also be referred to as chloride form, nitrate form, acetate form, carbonate form, etc., depending on the difference in its interlayer anions.
In Formula (1), x represents real number of typically 0.17 or more, preferably 0.19 or more, and typically 0.38 or less, preferably 0.36 or less. When x is below these lower limits, divalent metal hydroxide byproducts may occur, while when x is above these upper limits, trivalent metal hydroxide byproducts may occur, both of which may cause a decrease in productivity efficiency.
In Formula (1), m represents a real number of 0 or more but 2 or less.
The compositional makeup of a general hydrotalcite that satisfies the above formula can be determined by, e.g., the method described in the Examples section below.

*Surface treatment and the ratio of the surface treatment agent (requirement (A)):

The surface-treated hydrotalcite of the present invention is prepared by surface-treating a hydrotalcite having a compositional makeup represented by Formula (1). The term "surface treatment" herein refers to a treatment in which a surface treatment agent is chemically bound to hydrotalcite. The surface treatment agent bound to the surface of hydrotalcite particles is not washed away even if washed with deionized water.
In this disclosure, the surface treatment agents that can be used for the surface treatment of hydrotalcite may be, without limitation, at least one or more selected from the group consisting of anionic surfactants, cationic surfactants, phosphoric acid ester-based treatment agents, silane coupling agents, titanate coupling agents, aluminum coupling agents, silicone-based treatment agents, silicates, liquid glass, and polyanionic polymer compounds. Among them, from the viewpoint of efficient chemical bonding of the surface treatment agent to the particle surface of hydrotalcite, it is preferable to use a surface treatment agent with anionic functional groups, and from the viewpoint of improving dispersion, it is preferable to use a hydrophilic polymer as the surface treatment agent. Therefore, a polyanionic hydrophilic polymer compound is more preferred as a surface treatment agent.

The surface-treated hydrotalcite of the present invention is characterized in that a significantly larger amount of the surface treatment agent is introduced into the surface of the hydrotalcite particles compared to conventional products. Specifically, the mass ratio of the surface treatment agent to the hydrotalcite of the present invention is within the range of typically 20 mass % or higher, preferably 23 mass % or higher, more preferably 25 mass % or higher, and typically 50 mass % or lower, preferably 47 mass % or lower, more preferably 43 mass % or lower. Surface treatment of hydrotalcite has conventionally been well known, for example, as described in Patent Literature 1 (WO2018/169019 A). Although this document claims that a surface treatment agent can be introduced at any given mass ratio, the amount of a surface treatment agent actually introduced in the examples was only about 10 mass %. When a hydrotalcite is blended with a resin as described in Patent Literature 1, the amount of a surface treatment agent to be introduced has to be minimal for ensuring that the hydrotalcite particles are dispersed in the resin, because too large a ratio of the surface treatment agent will damage the physical properties of the resin. Therefore, the technical concept of introducing a significantly large amount of a surface treatment agent, such as 20 mass % or more relative to hydrotalcite, cannot be envisioned from the conventional technology.

The method and conditions for the surface treatment of the hydrotalcite of the present invention using the aforementioned surface treatment agent are not limited, and any method and conditions can be employed. In general, it is sufficient to mix the hydrotalcite and the surface treatment agent (usually in the presence of a liquid medium). However, from the viewpoint of chemically bonding a larger amount of the surface treatment agent to the particle surface of the hydrotalcite more efficiently than in the prior art, it is preferable to use a method that allows more efficient interaction between the hydrotalcite and the surface treatment agent (e.g., high-pressure homogenizer). It is also preferable to use an excess amount (e.g., typically 1.1 times or more, especially 1.2 times or more, by mass relative to the desired introduction amount). Other methods and conditions are discussed below.

The amount of a surface treatment agent in a general surface-treated hydrotalcite can be determined by, e.g., the method described in the Examples section below.

*Average width of primary particles (requirement (B)):

A characteristic of the surface-treated hydrotalcite of the present invention is that the average width of primary particles is within a predetermined range. The term "primary particle" herein refers to a particle with a definite boundary that cannot be further divided geometrically. Specifically, as shown in the schematic diagram of Figure 1 for example, when a primary particle of hydrotalcite crystal is assumed to be a hexagonal platelet, the long diameter measured as the length of the diagonal sides of the hexagon is defined as the width of the primary particle (W1). The arithmetic average of the widths measured for any 100 primary particles of hydrotalcite crystal is determined as the average width of the primary particles.

The average width of primary particles of the surface-treated hydrotalcite of the present invention is within the range of typically 5 nm or more, preferably 10 nm or more, more preferably 15 nm or more, and typically 120 nm or less, preferably 110 nm or less, more preferably 100 nm or less. If the average width of the primary particles is below these lower limits, dispersibility may deteriorate due to agglomeration, while if the average width of the primary particles exceeds these upper limits, dispersibility may also deteriorate, and the particles may be too large to meet the desired applications. Specifically, when the surface-treated hydrotalcite of the present invention is used in a drug delivery system, especially for antitumor drugs, if the average width of the primary particles is below the lower limits mentioned above, the drug may be more susceptible to renal ejection, whereby the drug retention in the body may be inhibited. On the other hand, if the average width of the primary particles exceeds the upper limits mentioned above, the EPR effect may be less effective, whereby drug accumulation in the tumor tissue may be inhibited.

A specific example of the method for determining the average width of primary particles of hydrotalcite includes: observing the hydrotalcite to be measured using, e.g., a transmission electron microscope (TEM); measuring the length diameters of any 100 primary particles are measured; and calculating the arithmetic average of these measurements as the average width of the primary particles. A sample for observation can be prepared by dropping a suspension containing hydrotalcite onto a sample table for observation, followed by air-drying or vacuum-drying. Alternatively, the average width of primary particles of hydrotalcite can also be determined using a scanning electron microscope (SEM) instead of a TEM, following substantially the same procedure. The magnification for measurement by TEM or SEM is not limited, but can be, e.g., 20,000 to 50,000 times.

*Monodispersity (requirement (C)):

A characteristic of the surface-treated hydrotalcite of the present invention is that the monodispersity represented by Formula (2) below satisfies a predetermined lower limit or higher.

[Formula 4]

$$\text{Monodispersity (\%)} = \frac{\text{Average width of primary particles}}{\text{Average diameter } (D_{50}) \text{ in suspension}} \times 100 \qquad (2)$$

In Formula (2), the "Average width of primary particles" is as described above, while the "Average particle size in suspension" is measured, e.g., by the following procedure. The surface-treated hydrotalcite is dispersed in a liquid medium (which refers to deionized water, unless otherwise specified) to prepare a suspension at a concentration of 50 g/L or lower. When the suspension is prepared, the surface-treated hydrotalcite should be dispersed in the liquid medium by agitation only, i.e., without carrying out any dispersion-facilitating treatment such as ultrasonication. The suspension thus obtained is then subjected to the measurement for cumulative 50% particle diameter ($D_{50}$) on a volume basis using a dynamic light scattering particle size distribution analyzer, and the measurement value can be used as the average particle diameter in the suspension.

The surface-treated hydrotalcite of the present invention is characterized in that the monodispersity represented by Formula (2) below satisfies a predetermined lower limit or higher. Specifically, the monodispersity of the surface-treated hydrotalcite of the present invention is typically 75% or higher, preferably 80% or higher, more preferably 82% or higher, still more preferably 85% or higher. This means that the surface-treated hydrotalcite of the present invention has excellent dispersibility when prepared in the form of suspension.

That is, as mentioned above, conventional nano-sized hydrotalcite particles show good dispersibility in water, but cannot maintain their dispersibility due to aggregation in the presence of salts, such as under biological conditions. In addition, when functional molecules are intercalated between the hydrotalcite layers, agglomeration occurs due to fusion of the particle surfaces, making it difficult to maintain their nanoparticle size. In contrast, the surface-treated hydrotalcite of the present invention disperses easily in a liquid medium and is difficult to aggregate, making it possible to prepare a suspension in which its fine particle size is maintained. Such excellent dispersibility is advantageous when using the surface-treated hydrotalcite of the present invention for various applications such as drug (functional molecule) delivery systems, as will be described below.

The surface-treated hydrotalcite of the present invention has only to satisfy the predetermined range of monodispersity represented by Formula (2) at least in a suspension using deionized water as the liquid medium, but may preferably satisfy the predetermined range of monodispersity represented by Formula (2) also in a suspension using other liquid medium. It is particularly preferable that the surface-treated hydrotalcite of the present invention has only to satisfy the predetermined range of monodispersity represented by Formula (2) both in a suspension using deionized water as the liquid medium and in a suspension using a physiological isotonic solution.

The cumulative 50% particle diameter ($D_{50}$) on a volume basis of the surface-treated hydrotalcite of the present invention is not restricted, as long as the monodispersity of Formula (2) above satisfies the predetermined range mentioned above in relation to the average width of the primary particles. For example, the diameter may preferably be 120 nm or less, particularly 110 nm or less, more particularly 100 nm or less. If the $D_{50}$ exceeds the upper limits mentioned above, the EPR effect may not be achieved, preventing the drug accumulation in the tumor tissue. The lower limit is not particularly limited, but may be typically 5 nm or more, especially 10 nm or more, and even 15 nm or more. If $D_{50}$ is below the lower limits, it may be susceptible to renal elimination, preventing the drug retention in the body.

* Suspension of the surface-treated hydrotalcite:

The surface-treated hydrotalcite of the present invention described above can be dispersed in a liquid medium to form a suspension. As mentioned above, the surface-treated hydrotalcite of the present invention has excellent dispersibility and is difficult to agglomerate, making it possible to prepare a suspension in which its fine particle size is maintained. Such a suspension in which the surface-treated hydrotalcite of the present invention is suspended in a liquid medium (hereinafter referred to as "the suspension of the present invention" as appropriate) is also encompassed by one of the aspects of the present invention.

The liquid medium used for the suspension of the present invention is not limited and can be selected depending on, e.g., the uses of the suspension, but may typically be deionized water or a physiological isotonic solution. The term "physiological isotonic solution" herein refers to a solution having an osmotic pressure equivalent to the in vivo osmotic pressure. Examples include normal saline, Ringer's solution, and phosphate buffered saline (PBS).

*Functional molecules and their retention ratio:

The surface-treated hydrotalcite of the present invention and its suspension may contain functional molecules between the layers of the hydrotalcite. In this case, the surface-treated hydrotalcite takes the form in which some of its interlayer anions $A^{n-}$ are ion-exchanged to the functional molecules. However, some of the functional molecules may be present outside the hydrotalcite layers (in the case of a suspension, in the liquid medium).

The functional molecules are not limited, and may be selected according to the uses of the surface-treated hydrotalcite or its suspension containing such functional molecules. Examples of functional molecules include, but are not limited to, drugs, antibiotics, antimicrobials, amino acids, peptides, proteins, therapeutic agents, hormones, enzymes, growth factors, RNAs, DNAs, and oligonucleotides. However, from the viewpoint of supportability between surface-treated hydrotalcite layers, functional molecules with anionic functional groups are preferred. The molecular weight of the functional molecule may typically be 1500 or less, especially 1000 or less, more particularly 500 or less. If the molecular weight exceeds the aforementioned upper limits, it may become difficult to intercalate the functional molecules, causing a decrease in the productivity.

However, especially when the suspension of the surface-treated hydrotalcite (i.e., the suspension of the present invention) contains functional molecules, the ratio of the functional molecules retained between the layers of the surface-treated hydrotalcite (functional molecule retention ratio) may preferably satisfy a predetermined lower limit or higher. Specifically, the functional molecule retention ratio of the suspension of the present invention can be determined in accordance with Formula (3) below.

[Formula 5]

$$\text{Functional molecule retention ratio (\%)} = \frac{\text{Mass of the functional molecules present between layers of surface-treated hydrotalcite}}{\text{Total mass of the functional molecules present in the suspension}} \times 100 \quad (3)$$

The functional molecule retention ratio in the suspension of the present invention depends on its liquid medium. For example, when the liquid medium is deionized water, the functional molecule retention ratio in the suspension of the invention may preferably be typically 75% or higher, especially 80% or higher, more particularly 85% or higher. When the liquid medium is a physiological isotonic solution, the functional molecule retention ratio in the suspension of the invention may preferably be typically 30% or higher, particularly 40% or higher, more particularly 50% or higher. Such excellent functional molecule retention is advantageous when the suspension of the present invention is used for various applications such as drug (functional molecule) delivery systems, as will be described below.

[II. Production method for the surface-treated hydrotalcite and its suspension]

The method for producing the surface-treated hydrotalcite of the present invention and its suspension (the suspension of the present invention) as described above is not particularly restricted. Typically, they can be produced by preparing a hydrotalcite satisfying the compositional makeup of Formula (1) mentioned above, surface-treating the hydrotalcite with an amount of surface treatment agent that satisfies the ratio mentioned above, and adjusting the particle size to satisfy the average width of the primary particles mentioned above.

The following description will be made on two examples of methods for producing the surface-treated hydrotalcite of the present invention and its suspension containing functional molecules intercalated between the hydrotalcite layers: one is a method involving aging the hydrotalcite in the chloride ($Cl^-$) form, nitrate ($NO_3^-$) form, or acetate ($CH_3COO^-$) form (hereinafter also referred to as "Production Method A"), while the other is a method involving ion-exchanging the hydrotalcite into carbonate ($CO_3^{2-}$) form followed by aging (hereinafter also referred to as "Production Method B"). However, these are just for the purpose of examples, and the methods for producing the surface-treated hydrotalcite of the present invention and its suspension are not limited to the methods consisting the steps described below, but can be implemented by adding, deleting, or modifying any steps.

<Production Method A: an embodiment in which hydrotalcite is aged in the chloride form, nitrate form, or acetate form>

Production Method A includes the steps of (1) preparation of raw materials, (2) reaction, (3) washing, (4) aging, (5) dispersion and surface treatment, (6) introduction of functional molecules, and (7) re-dispersion treatment.

(1) Raw material preparation step

In this step, aqueous solution of water-soluble complex metal salts and aqueous solution of alkali metal hydroxides are prepared as raw materials for hydrotalcite.

The aqueous solution of water-soluble complex metal salts may be prepared by dissolving divalent metal salts, trivalent metal salts, and monovalent organic acids and/or organic acid salts (complexing agents) that form complexes with trivalent metals into an aqueous solvent.

Examples of divalent metal salts include magnesium chloride, magnesium nitrate, magnesium acetate, zinc chloride, zinc nitrate, and zinc acetate. Examples of trivalent metal salts include aluminum chloride, aluminum nitrate, and aluminum acetate. The concentration of divalent metals in aqueous solution may be in the range of from 0.01 to 2 mol/L, and the concentration of trivalent metals can be in the range of 0.01 to 2 mol/L. The molar ratio of divalent metal ($M^{2+}$) to trivalent metal ($M^{3+}$) ($M^{2+}/M^{3+}$) may typically be 1.78 or higher, especially 1.94 or higher, and may typically be 4.88 or lower, especially 4.26 or lower. If the molar ratio ($M^{2+}/M^{3+}$) is below the lower limits, trivalent metal hydroxide byproducts may occur, while if it exceeds the upper limits, divalent metal hydroxides may occur, both of which may cause a decrease in production efficiency.

Examples of monovalent organic acids and/or organic acid salts that form complexes with trivalent metals, which serve as forming agents, include lactic acid, sodium lactate, formic acid, sodium formate, acetic acid, and sodium acetate. The amount of the complexing agent added may typically be 0.1 equivalent or more and 2.2 equivalents or less with respect to the amount of the trivalent metals. If the amount of the complexing agent added is less than the lower limits mentioned above, the average width of the primary particles of hydrotalcite may become too large. On the other hand, if the amount of the complexing agent added exceeds the upper limits mentioned above, anions from the complexing agent may enter between the layers of hydrotalcite, causing the suspension to gel due to swelling effects.

The aqueous alkali metal hydroxide solution may be prepared by dissolving alkali metal hydroxides into water. Examples of alkali metal hydroxides include sodium hydroxide and potassium hydroxide. The concentration of alkali metal hydroxide may typically be in the range of 0.01 mol/L or higher and 4 mol/L or lower.

(2) Generation reaction step

In this step, the aqueous solution of water-soluble complex metal salts and the aqueous solution of alkali metal hydroxides prepared in step (1) above are reacted continuously to produce hydrotalcite, whereby a suspension containing the hydrotalcite is obtained.

The concentration of the raw materials in the reaction solution at this step is not limited, but may be adjusted such that the hydrotalcite equivalent is in the range of 0.1 g/L or higher but 300 g/L or lower. The reaction temperature is also not limited, but may preferably be 0°C or higher but 60°C or lower. If the reaction temperature is lower than these lower limits, the suspension may freeze, while if the reaction temperature is higher than these upper limits, the average width of the primary particles may become too large. The pH during the reaction is also not limited, but may typically be within the range of pH 8.5 or higher but pH 11.5 or lower. If the reaction pH is lower than the lower limits, trivalent metal hydroxides may precipitate, while if the reaction pH is higher than the upper limits, the reaction product may agglomerate, resulting in poor dispersibility.

(3) Washing step

In this step, the suspension containing hydrotalcite obtained at step (2) above is washed with deionized water by dehydrating it, re-suspending it in deionized water, and dehydrating it again. This washing with deionized water removes salts such as sodium and prevents agglomeration of primary particles of hydrotalcite.

The means for dehydration is not limited, and may be natural dehydration by placing the material in a well-drained area, but dehydration may be accelerated by means of suction filtration or other means. The amount of deionized water used for resuspension is not limited, but for example, about 20 to 30 times the amount of deionized water may be used relative to the amount of hydrotalcite.

(4) Aging step

In this step, the suspension containing hydrotalcite after washing obtained at step (3) above is agitated and maintained for a predetermined time under predetermined conditions to cause aging, which allows the primary particles to grow slightly, thereby alleviating agglomeration of the primary particles and obtaining a suspension in which the primary particles are well dispersed.

The concentration of the suspension during aging is not limited, but it may usually be 0.1 g/L or higher and usually less than 300 g/L. If the concentration of the suspension is less than the lower limits, productivity may become too low, while if the concentration of the suspension exceeds the upper limits, primary particles may agglomerate. The temperature during aging is also not limited, but may preferably be set at 0°C or higher but 100°C or lower. If the aging temperature is below the lower limits, the formation of hydrotalcite may not progress sufficiently, while if the aging temperature exceeds the upper limits, the primary particles of hydrotalcite that are formed may grow too much. The aging time is also not limited, but may typically be 1 hour or longer but 60 hours or shorter. If the aging time is less than the lower limits, dispersion may be insufficient, while if the aging time exceeds the upper limits, the agglomeration state may not change.

(5) Dispersion and surface treatment step

In this step, the suspension containing the aged hydrotalcite obtained at step (4) above is subjected to surface treatment using a surface treatment agent along with dispersion treatment. Through this treatment, the surface treatment agent is bound to the surface of hydrotalcite. This treatment makes it possible to prevent the occurrence of strong aggregation that otherwise may occur during the ion exchange described below.

The means for dispersion treatment is not limited, and any known dispersion method may be used. Examples include methods using dispersion equipment such as bead mills, high-pressure homogenizers, and ultrasonic dispersion devices. Among these, from the viewpoint of sufficient interaction between the hydrotalcite and the surface treatment agent, it is preferable to use a high-pressure homogenizer. During the dispersion treatment of the hydrotalcite using such a dispersion method, surface treatment can also be carried out simultaneously, by coexisting a surface treatment agent with the hydrotalcite. The type and the amount of the surface treatment agent used are as described above.

The conditions for dispersion and surface treatment vary depending on the dispersion method used and the selection of the surface treatment agent. The following is an example of the dispersion treatment using a high-pressure homogenizer. The temperature during the dispersion treatment is not limited, but may preferably be set at 0°C or higher and 100°C or lower. If the temperature during the treatment is less than the lower limits mentioned above, the particles may not be dispersed sufficiently, while if the temperature exceeds the upper limits mentioned above, the primary particles may grow. The pressure during the dispersion treatment is also not limited, but may preferably be set at 100 bar or higher and 1000 bar or lower. If the pressure during the treatment is less than the lower limits, strong agglomeration may not be suppressed, while if the pressure exceeds the upper limits, the primary particles may be damaged, and re-agglomeration may occur. The duration of the dispersion treatment is also not limited, but may preferably be 1 minute or longer and 60 minutes or shorter. If the treatment time is less than the lower limits, sufficient dispersion may not be achieved, while if the treatment time exceeds the upper limits, the primary particles may be damaged, and re-agglomeration may occur.

(6) Functional molecule introduction step

In this step, functional molecules are added to the suspension of surface-treated hydrotalcite obtained at step (5) above and maintained for a predetermined time with stirring to exchange interlayer anions, thereby introducing the functional molecules into between the layers of the hydrotalcite.

The examples of functional molecules are as described above. Among them, functional molecules having anionic functional groups are preferred. Although it depends on the selection of the functional molecules and the surface treatment agent, if the functional molecules are anionic, they may also be used in the form of salts with appropriate counter cations, such as sodium salts. The amount of the salts of functional molecules used is also not limited, and may be determined depending on conditions such as the selection and the amount of the functional molecules to be introduced and the selection of surface treatment agent. For example, the amount of the salts of functional molecules used may preferably be 0.1 equivalent or more and 1.5 equivalents or less to the anion exchange capacity of the surface-treated hydrotalcite. If the amount of the salts of functional molecules used is less than the lower limits mentioned above, ion exchange may be insufficient, while if the amount of the salts of functional molecules used exceeds the upper limits mentioned above, production efficiency may deteriorate.

The conditions during the treatment for introducing the functional molecules are not limited, but may be set, for example, as follows. The temperature during the treatment is not limited, but may preferably be set at 0°C or higher and 100°C or lower. If the temperature during the treatment is below the lower limits, it may not be possible to suppress the synthesis of strong agglomeration, while if the temperature during the treatment exceeds the upper limits, the primary particles may grow. The duration of the treatment is also not limited, but may preferably be 0.5 hours or longer and 48 hours or shorter. If the treatment time is less than the lower limits, a sufficient introduction rate of functional molecules may not be obtained, while if the treatment time exceeds the upper limits, sufficient introduction efficiency may not be obtained.

## (7) Re-dispersion treatment step

In this step, the suspension of the surface-treated hydrotalcite into which the functional molecules have been introduced between its layers at step (6) above is subjected to re-dispersion treatment. This treatment makes it possible to break up and prevent agglomeration of the surface treated hydrotalcite.

The means for re-dispersion is not limited, and any known method for dispersion may be used as in the dispersion treatment of step (5) above. Examples include methods using dispersion equipment such as bead mills, high-pressure homogenizers, and ultrasonic dispersion equipment. The conditions for dispersion treatment vary depending on the dispersion method used. The following is an example of the re-dispersion treatment using a high-pressure homogenizer. The temperature during the treatment is not restricted, but may preferably be set at 0°C or higher and at 100°C or lower. If the temperature during the treatment is less than the lower limits mentioned above, sufficient re-dispersion may not be achieved, while if the temperature exceeds the upper limits, the primary particles may be damaged and re-agglomeration may occur. The pressure during the treatment is also not limited, but may preferably be 100 bar or higher but 1000 bar or lower. If the pressure during the treatment is less than the lower limits, weak agglomeration may not be broken, while if the pressure during the treatment exceeds the upper limits, the primary particles may be damaged and re-agglomeration may occur. The duration of the re-dispersion treatment is also not limited, but may preferably be 1 minute or longer and 60 minutes or shorter. If the treatment time is less than the lower limits, the synthesis of strong agglomeration may not be suppressed, while if the treatment time exceeds the upper limits, the primary particles may be damaged and re-agglomeration may occur.

## (8) Others

The suspension after the re-dispersion treatment at step (7) may be used for the desired uses, but may be subjected to any post-treatment as appropriate. Examples of such post-treatment include converting the liquid medium to a physiological isotonic solution. For example, when converting the liquid medium to physiological saline, after the re-dispersion treatment at step (7), 0.2 mol/L NaCl solution is added to the obtained suspension of surface- treated hydrotalcite to adjust the NaCl concentration to 0.9 mass%.

## <Production Method B: an embodiment in which hydrotalcite is ion-exchanged into the carbonate form and aged>

Production Method B includes the steps of (1) preparation of raw materials, (2) reaction, (3) washing and ion exchange with carbonate ions, (4) aging, (5) dispersion and surface treatment, (6) introduction of functional molecules, and (7) re-dispersion treatment.

## (1) Raw material preparing step

This step is the same as step (1) of Production Method A explained above.

## (2) Generation reaction step

This step is the same as step (2) of Production Method A explained above.

## (3) Washing and carbonate ion exchange step

In this step, the suspension containing hydrotalcite obtained at step (2) is dehydrated, washed with deionized water, and the washed hydrotalcite is then ion-exchanged to interlayer anions to carbonate ions.

The step of dehydration of the hydrotalcite suspension and washing with deionized water is the same as in step (1) of Production Method A explained above.

Conversion of interlayer anions to carbonate ions is carried out, e.g., by adding sodium carbonate solution to the washed hydrotalcite cake, mixed, and left stand, or by re-suspending the washed hydrotalcite cake in deionized water and then adding sodium carbonate solution, mixed, and left stand. The equivalent amount of sodium carbonate used may preferably be adjusted at 1 equivalent or higher and 2.5 equivalents or lower relative to the anion exchange capacity of the hydrotalcite. If the amount of sodium carbonate used is less than the lower limits, ion exchange may be insufficient, while if the amount of sodium carbonate used exceeds the upper limits, a large amount of unreacted product may be generated, which may be undesirable for industrial use. The temperature during the ion exchange is not limited, but may preferably be in the range of 30°C or higher and usually less than 90°C. If the temperature during the treatment is below the lower limits, ion exchange may be insufficient, while if the temperature exceeds the upper limits, the structure of the hydrotalcite may change. The duration of the ion exchange is also not limited, but may preferably be 15 minutes or longer and 60 hours or shorter. If the treatment time is less than the lower limits, ion exchange may be insufficient, while if the treatment time exceeds the upper limits, ion exchange may equilibrate, which is not desirable for efficiency.

After the ion exchange treatment, it is preferable to remove excess sodium carbonate solution by filtration or other means, and then wash the ion-exchanged hydrotalcite again with deionized water. This removes sodium salts and other salts and prevents agglomeration of the primary particles. The method of washing with deionized water is the same as in step (3) of Production Method A explained above.

(4) Aging step

This step is the same as step (4) of Production Method A explained above.

(5) Dispersion, surface treatment and ion-exchange/acid-injection step

In this step, the suspension containing the aged hydrotalcite obtained at step (4) above is subjected to surface treatment with a surface treatment agent along with dispersion treatment. This treatment allows for the surface treatment agent to be bound to the hydrotalcite surface, to thereby suppress the synthesis of strong agglomeration (aggregate) that otherwise may occur during the ion exchange described below.

The means for the dispersion treatment is not limited, and any known dispersion treatment method can be used. The details are the same as the conditions for dispersion treatment at step (5) of Production Method A explained above.

It is preferable to divide the surface treatment into two stages, primary surface treatment and secondary surface treatment, and insert ion exchange treatment by acid injection in between, as described below.

Specifically, the aged hydrotalcite suspension is first subjected to surface treatment (primary surface treatment) with a portion of the surface treatment agent to be used (e.g., about 10 mass % relative to the total mass of the hydrotalcite). The time for the primary surface treatment is also not limited, but may preferably be 1 minute or longer and 60 minutes or shorter. If the treatment time is less than the lower limits mentioned above, the surface treatment agent may not be sufficiently introduced, while if the treatment time exceeds the upper limits, the primary particles may become bruised and re-agglomerated. The details are the same as the conditions for the surface treatment at step (5) of Production Method A explained above.

The ion exchange treatment by acid injection is a treatment in which an acid is poured into the suspension after primary surface treatment to carry out ion exchange, thereby replacing $CO_3^{2-}$ ions in the interlayer with $NO_3^-$ and $Cl^-$ ions. The selection of the acid is not limited, examples thereof including nitric acid and hydrochloric acid. The amount of the acid used is also not limited, but may preferably be adjusted such that the pH of the suspension is within the range of pH 3 or higher and pH 3.5 or lower. If the pH of the suspension is below the lower limits, the surface of the hydrotalcite particles may be lysed, while if the pH of the suspension exceeds the upper limits, ion exchange may not proceed sufficiently. The concentration of the acid to be injected is also not limited, but may preferably be 0.5 mol/L or higher and 2 mol/L or lower. If the acid concentration is less than the lower limits, the pH may not drop easily and the liquid volume may increase too much, while if the acid concentration exceeds the upper limits, a pH bias may occur in the suspension. The suspension after acid injection is centrifuged at 30,000 x g for 40 minutes at room temperature, for example. The centrifuged supernatant is collected, re-suspended in deionized water, and then washed with deionized water. This washing operation may preferably be repeated about four times, for example. The precipitate after washing is re-suspended in deionized water.

The re-suspended solution is again subjected to surface treatment (secondary surface treatment) with the remainder of the surface treatment agent to be used (the remainder after partial use in the primary surface treatment). The duration of the secondary surface treatment is not limited, but may preferably be 1 minute or longer and 60 minutes or shorter. If the treatment time is less than the lower limits mentioned above, the surface treatment agent may not be sufficiently introduced, while if the treatment time exceeds the upper limits, the primary particles may become bruised and re-agglomeration may occur. The details are the same as the conditions for the surface treatment at step (5) of Production Method A explained above.

(6) Functional molecule introduction step

This step is the same as step (6) of Production Method A explained above.

(7) Re-dispersion treatment step

This step is the same as step (7) of Production Method A explained above.

(8) Others

As in the case of Production Method A, The suspension after the re-dispersion treatment at step (7) may be used for the desired uses, but may be subjected to any post-treatment as appropriate. Examples of such post-treatment include converting the liquid medium to a physiological isotonic solution. The details are the same as those explained for Production Method A above.

<Modifications to Production Methods A and B>

The above description of Production Methods A and B are examples only, and the method of producing the surface-treated hydrotalcite and its suspension is not limited to the process described below, but can be implemented by adding, deleting, or modifying any steps.

For example, the ion exchange method is used as the means for introducing functional molecules into the interlayer of hydrotalcite in Production Methods A and B (step (6) in each of Production Methods A and B). The ion-exchange method has the advantage of producing a product with excellent crystallinity because the hydrotalcite maintains its layered structure during the incorporation of functional molecules. However, the means for introducing functional molecules is not limited to the ion exchange method, but may be any other method such as coprecipitation or reconstruction method, depending on the type of the functional molecules and the uses of the resulting hydrotalcite.

The coprecipitation method is a method involving mixing an aqueous solution containing target anions with the water-soluble complex metal salt solution and the alkali metal hydroxide solution at the synthesis step of hydrotalcite, to thereby synthesize hydrotalcite and, at the same time, incorporate the target anions to between the hydrotalcite layers. Using functional molecule anions as the target anions at this step allows for the interlayer incorporation of the functional molecules. The coprecipitation method enables direct incorporation of molecules with large molecular size, which are difficult to incorporate by the ion exchange method, but has the disadvantage that the crystallinity of the product is lower than other methods.

The reconstruction method is a method involving calcining hydrotalcite at about 500°C to form a magnesium-aluminum oxide solid solution, and then adding the solid solution to an aqueous solution containing desired guest molecules, to thereby reconstruct hydrotalcite into which the desired guest molecules have been incorporated between the hydrotalcite layers. Using functional molecules as the desired guest molecules at this step allows for the interlayer incorporation of the functional molecules. Although the reconstruction method has the disadvantage of being labor intensive, it has the advantage that uncharged organic compounds as functional molecules can be introduced, by neutralizing the positive charge of the basic layer with $OH^-$.

[III. Uses of the surface-treated hydrotalcite and its suspension]

The surface-treated hydrotalcite and its suspension (the suspension of the present invention) described above exhibit excellent dispersibility. That is, as mentioned above, conventional nano-sized hydrotalcite particles show good dispersibility in water, but tend to agglomerate and cannot maintain dispersibility in the presence of salts, such as under physiological conditions. In addition, when introducing functional molecules between the layers of hydrotalcite, agglomeration occurs due to fusion of the particle surfaces, making it difficult to maintain their nanoparticle size. In contrast, the surface-treated hydrotalcite of the present invention is easily dispersed and does not agglomerate, making it possible to prepare suspensions in which its fine particle size is maintained. The present invention also has the advantage of suppressing both strong aggregation during the ion exchange and weak agglomeration after the ion exchange, both of which otherwise may occur in the conventional manufacturing process of hydrotalcite. Furthermore, the surface-treated hydrotalcite of the present invention can maintain its high dispersibility in the suspension form, not only when the liquid medium is deionized water containing no such salts, but also when the liquid medium is a physiological isotonic solution containing various salts.

Because of this excellent dispersibility, the surface-treated hydrotalcite of the present invention can be suitably used for various uses. Although the applications of the surface-treated hydrotalcite of the present invention are not limited, it is particularly desirable to use the surface-treated hydrotalcite of the present invention as a drug delivery system, by loading a pharmaceutically active compound as the functional molecules between the hydrotalcite layers, because of its excellent dispersibility when preparing suspensions, its excellent retention rate of functional molecules when loaded between the layers, and its excellent accumulation in specific tissues such as tumor tissue. Such a functional molecule delivery system using the surface-treated hydrotalcite of the present invention or its suspension containing a pharmaceutically active compound as the interlayer functional molecules (hereinafter also referred to as "the drug delivery system of the present invention" as appropriate) also constitutes one aspect of the present invention.

The drug delivery system of the present invention can be made in various embodiments with no restriction on its details, as long as it contains the surface-treated hydrotalcite of the present invention or its suspension, and the surface-treated hydrotalcite contains a pharmaceutically active compound as functional molecules. For example, the selection of the pharmaceutically active compound used in the drug delivery system of the present invention is not limited and may be any compound, as long as it can be introduced and loaded as the functional molecules between the layers of the surface-treated hydrotalcite of the present invention. Specifically, since the surface-treated hydrotalcite of the present invention has a suitable size for causing the EPR effect (i.e., an average width primary particle width of from 5 to 120 nm), the surface-treated hydrotalcite of the present invention may preferably be used as a delivery system to tumor tissue by EPR effect, by loading an anti-tumor pharmaceutical active compound as the interlayer functional molecules. The anti-tumor pharmaceutical active compound to be used is not limited, but may preferably be a low molecular weight compound that can be carried in between the hydrotalcite layers.

Specific examples of such small molecule anti-tumor compounds include: antitumor antibiotics such as doxorubicin, epirubicin, daunorubicin, bleomycin, and echinomycin D; alkylating agents such as cyclophosphamide, ifosfamide, melphalan, busulfan, thiotepa, nimustine, ranimustine, dacarbazine, procarbazine, temozolomide, carmustine, streptozotocin, and bendamustine; platinum-based antitumor agents such as oxaliplatin, carboplatin, cisplatin, abd nedaplatin; aromatase inhibitors such as exemestane, anastrozole, and letrozole; microtubule polymerization inhibitors such as vincristine and vinblastine; microtubule depolymerization inhibitors such as docetaxel, nab-paclitaxel, and paclitaxel; pyrimidine metabolism inhibitors such as fluorouracil and flucytosine; nucleotide analogs such as gemcitabine, cytarabine, thioguanine, fludarabine phosphate, and cladribine; topoisomerase inhibitors such as irinotecan, nogitecan, and epipodof etoposide; nitrosoureas such as nimustine and dacarbazine; and folic acid metabolism antagonists such as methotrexate. The dosage of the pharmaceutically active compound, the amount in the dosage form, the type of target disease, the route of administration, the dosage form, and the administration plan are also arbitrary, and can be selected and combined in various manners according to various conditions.

Specifically, the drug delivery system of the present invention may be used for any route of administration without any limitation. For example, it may be administered either systemically or locally, and either orally or parenterally. In the case of parenteral administration, it may be intravenous, intramuscular, intrathecal, subcutaneous, sublingual, transrectal, transvaginal, ophthalmic, ear, nasal, inhalation, spray, or transdermal administration. The dosage form of the drug delivery system of the present invention is also not limited and may be any dosage form depending on the route of administration. For example, it may be a solid dosage form or a liquid or semi-liquid dosage form. Examples of solid dosage forms include powder, granules, tablets, and pills. Examples of liquid or semi-liquid dosage forms include oral liquids, syrups, and injectable solutions. Whatever the route of administration or dosage form, the drug delivery system of the present invention can be applied to achieve a suitable effect, as long as good dispersibility in an aqueous medium such as a physiological isotonic solution is required during its manufacture and/or use.

When the drug delivery system of the present invention is made into a solid formulation, e.g., by formulating the surface-treated hydrotalcite of the present invention containing a pharmaceutically active compound as interlayer functional molecules, along with other ingredients such as various excipients as needed. Alternatively, a suspension of the surface-treated hydrotalcite containing a pharmaceutically active compound as interlayer functional molecules can be formulated by adding other ingredients such as various excipients, and then removing the liquid medium by distillation, lyophilization, or other methods. On the other hand, when the drug delivery system of the present invention is made into a liquid or semi-liquid formulation, for example, by formulating the surface-treated hydrotalcite of the present invention containing a pharmaceutically active compound as interlayer functional molecules, along with other ingredients such as various excipients as needed.

Examples of other ingredients that can be used in the drug delivery system of the invention include, but are not limited to: excipients such as mannitol, erythritol, powdered reduced maltose water candy, crystalline cellulose, and corn starch; disintegrants such as crospovidone, croscarmellose sodium, sodium starch glycolate, partially alphalated starch, calcium carmellose, and low substituted hydroxypropyl cellulose; sweetening agents such as sucralose, aspartame, acesulfame potassium, sodium saccharin, monoammonium glycyrrhizate, and thaumatin; and other additives such as fluidizers, colorants, and fragrances. These ingredients may be selected and used as appropriate depending on the dosage form of the pharmaceutical composition of the present invention. The pharmaceutical composition of the present invention may contain either any one of these ingredients singly or any two or more of these ingredients in any combination and ratio.

The details of the drug delivery system of the present invention described above are only examples. A person skilled in the art can implement the drug delivery system of the present invention by selecting appropriate conditions, taking into consideration the known knowledge of pharmaceutical formulations, for example, University of the Sciences in Philadelphia, "Remington: The Science and Practice of Pharmacy, 20th EDITION "Remington: The Science and Practice of Pharmacy, 20th EDITION," Lippincott Williams & Wilkins, 2000, etc.

**EXAMPLES**

The invention will be described in more detail in the following examples, but these examples are presented herein only for the sake of explanation, and the present invention is not limited to these examples in any sense.

[I. Production of hydrotalcite suspension]

Samples of surface-treated hydrotalcite suspensions in Examples 1 to 5 and Comparative Examples 1 to 3 were produced by the following procedure.

*Example 1:

(Preparation of raw materials and synthesis reaction)

Magnesium chloride hexahydrate (FUJIFILM Wako Pure Chemicals) was used as a divalent metal salt, aluminum chloride hexahydrate (FUJIFILM Wako Pure Chemicals) as a trivalent metal salt, and sodium lactate (KISHIDA CHEMICAL Chemical) as a monovalent organic acid and/or an organic acid salt (complexing agent) that forms a complex with the trivalent metal, in 1.75 equivalents relative to the amount of aluminum. A water-soluble complex metal salt solution was prepared by dissolving these salts in deionized water such that the magnesium content is 0.86 mol/L, the aluminum content is 0.4 mol/L, and the lactic acid content is 0.7 mol/L. An alkali metal hydroxide solution was also prepared by using sodium hydroxide (FUJIFILM Wako Pure Chemicals) as an alkali metal hydroxide and dissolving it in deionized water at a concentration of 2.52 mol/L. Both the water-soluble complex metal salt solution and the alkali metal hydroxide solution were injected into a cylindrical reaction tank with an overflow capacity of 220 mL each at a flow rate of 110 mL/min, to thereby cause reaction to form hydrotalcite continuously at 25°C while stirring the tank. The flow rate of the alkali metal hydroxide solution was controlled such that the pH in the tank is adjusted to within a range of from 9.3 to 9.6. After the reaction, the resulting hydrotalcite suspension was dehydrated by suction filtration to form a hydrotalcite cake.

(Washing, aging, primary dispersion and surface treatment)

Deionized water was added to the resulting hydrotalcite cake at a volume 30 times the mass ratio, and the cake was washed with deionized water by suction filtration. The washed cake was then re-suspended in deionized water with adjusting the concentration to 60 g/L, and maintained at 60°C for 18 hours for aging. The resulting suspension of aged hydrotalcite was placed in a high-pressure homogenizer, and 60 mass% of MARIALIM HKM-50A (NOF) was added as a surface treatment agent (to 100 mass% hydrotalcite) to carry out surface treatment while dispersing at 900 bar and 25°C for 0.5 hour.

(Introduction of functional molecules, washing, and secondary dispersion treatment)

The resulting suspension of surface-treated hydrotalcite was combined with sodium folate as a salt of functional molecules in an amount of 1.0 equivalent to the anion exchange capacity of the hydrotalcite. The mixture was kept at 90°C for 18 hours with stirring to carry out ion exchange between the interlayer anions and the functional molecules, folic acid, to thereby introduce the functional molecules into between the hydrotalcite layers. The resulting suspension of surface-treated hydrotalcite containing interlayer functional molecules was then centrifuged to cause sedimentation of the surface-treated hydrotalcite containing interlayer functional molecules, and the supernatant was discarded to dehydrate the sedimentation. The resulting precipitate of surface-treated hydrotalcite containing interlayer functional molecules was mixed with 20 times the mass ratio of deionized water, re-suspended, and dehydrated again, and this washing process was repeated four times. The washed suspension was then placed in a high-pressure homogenizer and re-dispersed under conditions of 900 bar at 25°C for 1 hour, thereby producing the surface-treated hydrotalcite suspension of Example 1, which carries folic acid as functional molecules between the layers of hydrotalcite.

*Example 2:

The surface-treated hydrotalcite suspension of Example 2, which carries folic acid as functional molecules between the layers of hydrotalcite, was prepared in accordance with the same process as that of Example 1 except that a high-pressure homogenizer was not used during the primary dispersion and surface treatment, but instead the surface treatment was performed at 25°C for 0.5 hours with stirring and mixing.

*Example 3:

The surface-treated hydrotalcite suspension of Example 3, which carries folic acid as functional molecules between the layers of hydrotalcite, was prepared in accordance with the same process as that of Example 1 except that the heating temperature during aging was changed from 60°C to 90°C.

*Example 4:

(Preparation of raw materials, synthesis reaction, and ion exchange with carbonate ions)

The same procedure as in Example 1 was used for raw material preparation and hydrotalcite formation reaction to obtain a hydrotalcite cake. Sodium carbonate solution of 1.5 equivalents to the anion exchange capacity of hydrotalcite contained in the cake was injected into the cake to cause ion exchange, thereby introducing carbonate ions into between the layers of hydrotalcite.

(Aging, dispersion and surface treatment, ion exchange by acid injection)

The resulting hydrotalcite containing interlayer carbonate ions was aged according to the same procedure as in Example 1, and then subjected to primary surface treatment while being dispersed according to the same procedure as in Example 1, except that the amount of the surface treatment agent, MARIALIM HKM-50A (NOF), was changed to 24 mass% (relative to 100 mass% hydrotalcite). The resulting suspension of hydrotalcite containing interlayer carbonate ions having undergone the primary surface treatment was combined with 0.5 mol/L nitric acid in an amount of 2.2 equivalents to the anion exchange capacity of hydrotalcite with adjusting the pH to 3.0 to 3.3, thereby producing a suspension of hydrotalcite containing interlayer nitrate ions having undergone the primary surface treatment. This suspension was then centrifuged to cause sedimentation, and the supernatant was discarded to dehydrate the sedimentation. The resulting precipitate was mixed with 20 times the mass ratio of deionized water, re-suspended, and dehydrated again, and this washing process was repeated four times. The suspension of the washed hydrotalcite containing interlayer nitrate ions having undergone the primary surface treatment was placed in a high-pressure homogenizer. 48 mass% of MARIALIM HKM-50A (NOF) was added as a surface treatment agent (to 100 mass% hydrotalcite), and secondary surface treatment was performed while dispersing at 900 bar and 25°C for 0.5 hour.

(Introduction of functional molecules, washing, and re-dispersion treatment)

The resulting suspension of the hydrotalcite containing interlayer nitrate ions having undergone the secondary surface treatment was combined with sodium folate as a salt of functional molecules in an amount of 1.0 equivalent to the anion exchange capacity of the hydrotalcite. The mixture was kept at 60°C for 16 hours with stirring to carry out ion exchange between the interlayer anions and the functional molecules, folic acid, to thereby introduce the functional molecules into between the hydrotalcite layers. The resulting suspension of surface-treated hydrotalcite containing interlayer functional molecules was subjected to washing and re-dispersion treatment in accordance with the same procedure as in Example 1, thereby producing the surface-treated hydrotalcite suspension of Example 4, which carries folic acid as functional molecules between the layers of hydrotalcite.

*Example 5:

The surface-treated hydrotalcite suspension of Example 5, which carries methotrexate (MTX) as functional molecules between the layers of hydrotalcite, was prepared in accordance with the same process as that of Example 4 except the following. The heating temperature during aging was changed from 60°C to 90°C, the aging time was changed from 18 hours to 44 hours, and the amount of nitric acid used for ion exchange was changed to 1.5 equivalents to the anion exchange capacity of hydrotalcite. In addition, 1.2 equivalents of the sodium salt of methotrexate (MTX) was used instead of sodium folate as the salt of the functional molecules, the heating temperature during ion exchange to introduce the functional molecules was changed from 60°C to 90°C, and the retention time was changed from 16 hours to 18 hours..

*Comparative Examples 1:

The surface-treated hydrotalcite suspension of Comparative Example 1, which carries folic acid as functional molecules between the layers of hydrotalcite, was prepared in accordance with the same process as that of Example 1 except that the primary dispersion and surface treatment between the aging and the introduction of functional molecules was omitted.

*Comparative Examples 2:

The surface-treated hydrotalcite suspension of Comparative Example 2, which carries folic acid as functional molecules between the layers of hydrotalcite, was prepared in accordance with the same process as that of Example 1 except that the primary dispersion and surface treatment between the aging and the introduction of functional molecules was omitted, and that during the secondary dispersion treatment after introduction of functional molecules, surface treatment was also performed according to the same procedure as the primary dispersion and surface treatment in Example 1, followed by further centrifugal separation and washing treatment after introduction of functional molecules.

*Comparative Examples 3:

The surface-treated hydrotalcite suspension of Comparative Example 3, which carries folic acid as functional molecules between the layers of hydrotalcite, was prepared in accordance with the same process as that of Example 1 except the following. The heating temperature during aging was changed from 60°C to 90°C, the primary dispersion and surface treatment between the aging and the introduction of functional molecules was omitted, and the heating temperature during ion exchange to introduce functional molecules was changed from 90°C to 60°C and the holding time from 18 hours to 16 hours. Furthermore, during the secondary dispersion treatment after introduction of functional molecules, dispersion and surface treatment were performed according to the same procedure as the primary dispersion and surface treatment in Example 1, followed by centrifugal separation and washing treatment after introduction of the functional molecules.

[II. Analysis methods of the hydrotalcite suspension samples]

The suspensions of surface-treated hydrotalcite obtained in Examples 1 to 5 and Comparative Examples 1 to 3 were analyzed for various physical properties by the following procedures.

*Chemical composition analysis and quantitative determination of the surface treatment agent

The suspension of surface-treated hydrotalcite in each of Examples 1 to 5 and Comparative Examples 1 to 3 was centrifuged at 30,000 x g at room temperature for 40 minutes. The centrifugal supernatant was collected, and the precipitate was re-suspended in deionized water, and centrifuged again. This washing operation was repeated four times, whereby salts, dispersants, and other substances not bound to hydrotalcite were removed with the supernatant, while only the surface treatment agent bound to hydrotalcite remained in the precipitate fraction to be recovered. The precipitate fraction was washed and vacuum dried, and the resulting solids were ground with a mortar to prepare a powder sample.
The resulting powder sample was subjected to the chemical composition analysis and quantitative determination of the surface treatment agent in accordance with the following assay methods.

*Metal hydroxide: the sample was heated and dissolved in hydrochloric acid, the quantity of the metal ions in the metal hydroxide were determined by chelatometric titration, and the mass of the metal hydroxide was determined from the quantitated value.
*Interlayer moisture content: calculated from the mass loss up to 200°C using TG-DTA (model number: 2000SA, manufactured by BRUKERAXS Co., Ltd.) at a temperature increase rate of 10°C/min, with an atmosphere of 100 mL/min of air, and a sample volume of 10 mg.
*Functional molecules: the amount was determined by measuring absorbance using a UV-visible spectrophotometer (model number: U-4100, HITACHI). For example, it was determined by absorbance at 296 nm when the functional molecules were folic acid, and at 302 nm when the functional molecules were MTX (methotrexate).
*Lactic acid: the amount was determined by liquid chromatography using a high-performance liquid chromatograph (model number: Chromaster, HITACHI).
*$CO_3$: quantitated based on JIS.R.9101using an AGK-type CO2 simple and precise quantitative apparatus (Tsutsui Scientific Instruments Co., Ltd.).
*$NO_3$: quantitated based on JIS K.0102 45.1 according to the method for determination nitrate ions and nitrite ions in the total nitrogen determination methods.
*Cl: quantitated by ICP issuance spectrometry using an emission spectrometer (model number SPS3500DD, HITACHI).
*Amount of the surface treatment agent: the masses of metal hydroxides, interlayer water content, functional molecules, lactic acid, $CO_3$, $NO_3$, and Cl were subtracted from the mass of the powder sample, and the remaining mass was determined as the amount of the surface treatment agent.

*Suspension samples in deionized water and in normal saline:

Suspension samples of the surface-treated hydrotalcite in each of Examples 1 to 5 and Comparative Examples 1 to 3 was prepared using deionized water and normal saline. Specifically, the produced surface-treated hydrotalcite suspension was used as the deionized-water suspension sample. The normal-saline suspension sample was prepared by adding 0.2mol/L NaCl aqueous solution to the produced surface-treated hydrotalcite suspension to adjust the NaCl concentration to 0.9 mass %.

*Average width of primary particles:

Each of the deionized-water suspension sample and the normal-saline suspension sample of surface-treated hydrotalcite in each of Examples 1 to 5 and Comparative Examples 1 to 3 was dropped onto a sample stand for TEM observation, vacuum dried and prepared for observation, and observed by TEM at 20,000-50,000x magnification. The long diameters of any 100 primary particles of hydrotalcite crystals in the field of view were measured, and the arithmetic average was used as the average width of the primary particles.

*Average particle diameter in suspension by dynamic light scattering:

Each of the deionized-water suspension sample and the normal-saline suspension sample of surface-treated hydrotalcite in each of Examples 1 to 5 and Comparative Examples 1 to 3 was mixed with deionized water as appropriate to adjust the hydrotalcite mass equivalent concentration to 50 g/L or less. The cumulative 50% particle diameter ($D_{50}$) on a volume basis was then measured using a dynamic light scattering particle size analyzer (ELSZ2, Otsuka Electronics) and determined as the average particle diameter in suspension. Ultrasonic treatment was not performed during the measurement.

*Monodispersity:

Each of the deionized-water suspension sample and the normal-saline suspension sample of surface-treated hydrotalcite in each of Examples 1 to 5 and Comparative Examples 1 to 3 was subjected to the measurement of the monodispersity in accordance with Formula (2) below, based on the average width of primary particles and the average particle diameter in suspension ($D_{50}$).
[Formula 6]

$$\text{Monodispersity (\%)} = \frac{\text{Average width of primary particles}}{\text{Average diameter (}D_{50}\text{) in suspension}} \times 100 \qquad (2)$$

[III. Analysis results of the hydrotalcite suspension samples]

The production method, compositional makeup, and physical properties of the surface-treated hydrotalcite suspension of each of Examples 1 to 5 and Comparative Examples 1 to 3 are summarized in Table 1-1 and Table 1-2 below. In the tables, "HT" stands for hydrotalcite, "MTX" for methotrexate, and "h" for hours.

[Table 1-1]

| Table 1 | | | Example 1 | Example 2 | Example 3 | Example 4 | Example 5 |
|---|---|---|---|---|---|---|---|
| | Anions during aging | | $Cl^-$ | $Cl^-$ | $Cl^-$ | $CO_3^{2-}$ | $CO_3^{2-}$ |
| | Functional molecules | | Folic acid | Folic acid | Folic acid | Folic acid | MTX |
| | Aging before ion exchange | | 60°C,18h | 60°C,18h | 90°C,18h | 60°C,18h | 90°C,44h |
| Production method | Ion exchange equivalents | $CO_3 \rightarrow NO_3$ | - | - | - | 2.2 | 1.5 |
| | | $CO_3, NO_3 \rightarrow$ Folic acid, MTX | 1.0 | 1.0 | 1.0 | 1.0 | 1.2 |
| | Ion exchange conditions | | 90°C,18h | 90°C,18h | 90°C,18h | 60°C,16h | 90°C,18h |

(continued)

| Table 1 | | | Example 1 | Example 2 | Example 3 | Example 4 | Example 5 |
|---|---|---|---|---|---|---|---|
| Chemical composition | x in Formula (1) | | 0.33 | 0.33 | 0.33 | 0.38 | 0.36 |
| | Ratio of folic acid or MTX in $(A^{n-})_{x/n}$ in Formula (1) (%) | | 50 | 46 | 47 | 50 | 60 |
| | m in Formula (1) | | 1.5 | 1.3 | 1.4 | 1.1 | 1.3 |
| | Mass ratio of surface treatment agent to hydrotalcite (%) | | 33 | 34 | 34 | 20 | 26 |
| Properties | Average width of primary particles (nm) | | 53 | 51 | 85 | 44 | 80 |
| | Deionized-water suspension sample | $D_{50}$ (volume distribution) (nm) | 58 | 49 | 75 | 49 | 90 |
| | | Monodispersity [{(Average width of primary particles) ÷ $D_{50}$} x 100] (%) | 91 | 104 | 113 | 90 | 89 |
| | Normal-saline suspension sample | $D_{50}$ (volume distribution) (nm) | 68 | 63 | 69 | 49 | 92 |
| | | Monodispersity [{(Average width of primary particles) ÷ $D_{50}$} x 100] (%) | 78 | 81 | 123 | 90 | 87 |

[Table 1-2]

| Table 1 | | | Comparative Example 1 | Comparative Example 2 | Comparative Example 3 |
|---|---|---|---|---|---|
| Production method | Anions during aging | | $Cl^-$ | $Cl^-$ | $Cl^-$ |
| | Functional molecules | | Folic acid | Folic acid | Folic acid |
| | Aging before ion exchange | | 60°C,18h | 60°C,18h | 90°C,18h |
| | Ion exchange equivalents | $CO_3 \rightarrow NO_3$ | - | - | - |
| | | $CO_3, NO_3 \rightarrow$ Folic acid, MTX | 1.0 | 1.0 | 1.0 |
| | Ion exchange conditions | | 90°C,18h | 90°C,18h | 60°C,16h |
| Chemical composition | x in Formula (1) | | 0.32 | 0.33 | 0.33 |
| | Ratio of folic acid or MTX in $(A^{n-})_{x/n}$ in Formula (1) (%) | | 60 | 57 | 43 |
| | m in Formula (1) | | 1.2 | 1.2 | 1.2 |
| | Mass ratio of surface treatment agent to hydrotalcite (%) | | 0 | 7 | 14 |

(continued)

| Table 1 | | | Comparative Example 1 | Comparative Example 2 | Comparative Example 3 |
|---|---|---|---|---|---|
| Properties | | Average width of primary particles (nm) | 87 | 88 | 76 |
| | Deionized -water suspension sample | $D_{50}$ (volume distribution) (nm) | 6448 | 166 | 131 |
| | | Monodispersity [{(Average width of primary particles) - $D_{50}$} x 100] (%) | 1 | 53 | 58 |
| | Normal -saline suspension sample | $D_{50}$ (volume distribution) (nm) | 6432 | 155 | 142 |
| | | Monodispersity [{(Average width of primary particles) - $D_{50}$} x 100] (%) | 1 | 57 | 54 |

## INDUSTRIAL APPLICABILITY

The surface-treated hydrotalcite of the present invention and its suspension have excellent dispersibility in liquid media such as physiological isotonic solution, and can retain functional molecules between the layers with high retention rate, and therefore can be used as a drug delivery system by retaining drugs as functional molecules, and can be widely applied to the technical fields of medicine, chemistry, engineering, etc. Thus, the present invention has a very high utility value.

## Claims

1.  A surface-treated hydrotalcite comprising a hydrotalcite with a compositional makeup represented by formula (1) below treated with a surface treatment agent, wherein the surface-treated hydrotalcite satisfies the requirements (A) to (C) below.
    [Formula 1]

$$(M^{2+})_{(1-x)}(M^{3+})_x(OH)_2(A^{n-})_{x/n} \cdot mH_2O \qquad (1)$$

In Formula (1), $M^{2+}$ represents at least one divalent metal cation, $M^{3+}$ represents at least one trivalent metal cation, $A^{n-}$ represents at least one n-valent anion, n represents an integer of 1 to 6, x represents a real number satisfying $0.17 \leq x \leq 0.38$, and m represents a real number satisfying $0 \leq m \leq 2$.

(A) The mass ratio of the surface treatment agent to the hydrotalcite is 20 mass % or higher but 50 mass % or lower.
(B) The average width of the primary particles is from 5 nm to 120 nm.
(C) The monodispersity represented by Formula (2) below is 75% or higher.

[Formula 2]

$$\text{Monodispersity (\%)} = \frac{\text{Average width of primary particles}}{\text{Average diameter } (D_{50}) \text{ in suspension}} \times 100 \qquad (2)$$

2.  The surface-treated hydrotalcite according to claim 1, wherein the average particle diameter $(D_{50})$ of the hydrotalcite in a suspension is 120 nm or less.

3.  The surface-treated hydrotalcite according to claim 1 or 2, wherein the surface treatment agent is at least one selected from the group consisting of anionic surfactants, cationic surfactants, phosphoric acid ester-based treatment agents, silane coupling agents, titanate coupling agents, aluminum coupling agents, silicone-based treatment agents, silicates, liquid glass, and polyanionic polymer compounds.

4. The surface-treated hydrotalcite according to any one of claims 1 to 3, further comprising a functional molecule between layers.

5. The surface-treated hydrotalcite according to claim 4, wherein the functional molecule is at least one selected from the group consisting of drugs, antibiotics, antimicrobials, amino acids, peptides, proteins, therapeutic agents, hormones, enzymes, growth factors, RNAs, DNAs, and oligonucleotides.

6. A surface-treated hydrotalcite suspension comprising a liquid medium and a surface-treated hydrotalcite according to any one of claims 1 to 3 suspended in the liquid medium.

7. The surface-treated hydrotalcite suspension according to claim 6, wherein the liquid medium is deionized water or a physiological isotonic solution.

8. The surface-treated hydrotalcite suspension according to claim 6 or 7, wherein the liquid medium is a physiological isotonic solution, and the monodispersity of the surface-treated hydrotalcite in the physiological isotonic solution is 75% or higher.

9. The surface-treated hydrotalcite suspension according to any one of claims 6 to 8, further comprising a functional molecule between layers of the surface-treated hydrotalcite.

10. The surface-treated hydrotalcite suspension according to claim 9, wherein the functional molecule is at least one selected from the group consisting of drugs, antibiotics, antimicrobials, amino acids, peptides, proteins, therapeutic agents, hormones, enzymes, growth factors, RNAs, DNAs, and oligonucleotides.

11. A functional molecule delivery system comprising a surface-treated hydrotalcite according to any one of claims 1 to 5 and/or a surface-treated hydrotalcite suspension according to any one of claims 6 to 10.

FIGURE 1

Width of primary particles ($W_1$)

## INTERNATIONAL SEARCH REPORT

| International application No. |
|---|
| **PCT/JP2021/031394** |

### A. CLASSIFICATION OF SUBJECT MATTER

*C01F 7/00*(2006.01)i; *A61K 9/14*(2006.01)i; *A61K 31/198*(2006.01)i; *A61K 38/22*(2006.01)i; *A61K 45/00*(2006.01)i; *A61K 47/52*(2017.01)i; *A61P 3/02*(2006.01)i; *A61P 31/04*(2006.01)i; *A61P 35/00*(2006.01)i; *A61P 43/00*(2006.01)i; *B82Y 5/00*(2011.01)i; *B82Y 30/00*(2011.01)i

FI: C01F7/00 C; A61K9/14; A61K47/52; A61P31/04; A61P43/00 111; A61P3/02; A61K45/00; A61K38/22; A61K31/198; B82Y5/00; B82Y30/00; A61P35/00

According to International Patent Classification (IPC) or to both national classification and IPC

### B. FIELDS SEARCHED

Minimum documentation searched (classification system followed by classification symbols)

C01F7/00; A61K9/14; A61K31/198; A61K38/22; A61K45/00; A61K47/52; A61P3/02; A61P31/04; A61P35/00; A61P43/00; B82Y5/00; B82Y30/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2018/117114 A1 (KYOWA CHEMICAL INDUSTRY CO., LTD) 28 June 2018 (2018-06-28) entire text | 1-11 |
| A | WO 2016/174987 A1 (KYOWA CHEM IND CO LTD) 03 November 2016 (2016-11-03) entire text | 1-11 |
| A | WO 2013/147284 A1 (KYOWA CHEMICAL INDUSTRY CO., LTD) 03 October 2013 (2013-10-03) entire text | 1-11 |
| A | WO 2018/062360 A1 (UNIV NAT CORP EHIME UNIV) 05 April 2018 (2018-04-05) entire text | 1-11 |
| A | JP 2018-184305 A (MARUO CALCIUM) 22 November 2018 (2018-11-22) entire text | 1-11 |

☑ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **20 October 2021** | **02 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| | International application No. |
|---|---|
| | **PCT/JP2021/031394** |

**C.** **DOCUMENTS CONSIDERED TO BE RELEVANT**

| Category* | Citation of document, with indication, where appropriate, of the relevant passages | Relevant to claim No. |
|---|---|---|
| A | JP 2018-184306 A (MARUO CALCIUM) 22 November 2018 (2018-11-22)<br>    entire text | 1-11 |
| A | WO 2015/068312 A1 (KYOWA CHEM IND CO LTD) 14 May 2015 (2015-05-14)<br>    entire text | 1-11 |

Form PCT/ISA/210 (second sheet) (January 2015)

## INTERNATIONAL SEARCH REPORT
### Information on patent family members

International application No.

**PCT/JP2021/031394**

| Patent document cited in search report | | | Publication date (day/month/year) | Patent family member(s) | | | Publication date (day/month/year) |
|---|---|---|---|---|---|---|---|
| WO | 2018/117114 | A1 | 28 June 2018 | US entire text EP entire text | 2020-0087157 003560893 | A1 A1 | |
| WO | 2016/174987 | A1 | 03 November 2016 | (Family: none) | | | |
| WO | 2013/147284 | A1 | 03 October 2013 | US entire text EP entire text CN KR | 2015-0010652 002832695 104169219 10-2014-0138628 | A1 A1 A A | |
| WO | 2018/062360 | A1 | 05 April 2018 | (Family: none) | | | |
| JP | 2018-184305 | A | 22 November 2018 | (Family: none) | | | |
| JP | 2018-184306 | A | 22 November 2018 | (Family: none) | | | |
| WO | 2015/068312 | A1 | 14 May 2015 | US entire text EP entire text CN KR | 2016-0227715 003067386 105683273 10-2016-0089334 | A1 A1 A A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018169019 A

- JP 2007538098 A

**Non-patent literature cited in the description**

- **MATSUMURA et al.** *Cancer Research,* 1986, vol. 46 (12), 6387-6392
- Remington: The Science and Practice of Pharmacy. University of the Sciences in Philadelphia

- Remington: The Science and Practice of Pharmacy. Lippincott Williams & Wilkins, 2000